# EUROPEAN PATENT SPECIFICATION

(11) **EP 3 416 953 B1**
(45) Date of publication and mention of the grant of the patent: **30.09.2020**
(21) Application number: 17701154.1
(22) Date of filing: 25.01.2017
(51) Int. Cl.: C07D 251/60, C07C 273/12

(54) **METHOD FOR REVAMPING A HIGH PRESSURE MELAMINE PLANT**
VERFAHREN ZUR MODERNISIERUNG EINER HOCHDRÜCKMELAMINANLAGE
PROCÉDÉ POUR MODERNISER UNE INSTALLATION DE PRODUCTION DE MÉLAMINE SOUS HAUTE PRESSION

(30) Priority: 19.02.2016 EP 16156505
(43) Date of publication of application: 26.12.2018
(60) Divisional application: 19160019.6
(73) Proprietor: Casale SA, 6900 Lugano (CH)
(72) Inventor: DI CARLO, Gabriele, 6900 Lugano (CH); SCOTTO, Andrea, 6932 Breganzona (CH); GAMBA, Simone, 24040 Pagazzano (BG) (IT)
(74) Representative: M. Zardi & Co S.A.
(86) International application number: PCT/EP2017/051478
(87) International publication number: WO 2017/140465

(56) References cited:
- EP-A1- 1 054 006
- WO-A1-02/14289
- WO-A2-2009/080176

## Description

### Field of application

The invention relates to the field of melamine production from urea and relates in particular to the purification section of a high pressure melamine plant and a related process.

### Prior Art

The processes for the synthesis of melamine from urea comprise low pressure catalytic processes and high pressure non-catalytic processes, typically above 7 MPa. These processes are well-known in the art.

A conventional type of plant for the synthesis of melamine using the high pressure non-catalytic process comprises a high pressure section substantially limited to a reactor, called melamine reactor, wherein the urea melt feed is converted into raw melamine with addition of heat.

Conversion of urea into melamine also generates gases mainly consisting of ammonia and carbon dioxide (so-called "off-gas") and a number of by-products (mainly OATs and polycondensates). The reaction products (melamine and off-gas), the by-products and the unreacted urea are further processed in a purification section operating at significantly lower pressure and temperature and generally comprising a quencher, a stripper and an absorber.

In the quencher, raw melamine is dissolved in an ammonia aqueous solution to separate off-gas from melamine. The off-gas are conveyed to a condensation section and melamine is supplied to the stripper, preferably a steam stripper, for removal of residual off-gas. The latter are absorbed in water inside the absorber and melamine is subjected to further purification in downstream equipment.

This configuration is widely used, but has a number of drawbacks.

Generally, the melamine plant is combined with a urea plant which produces the urea melt feed, starting from NH₃ and CO₂. The off-gas extracted from the quencher of the melamine plant are thus generally recycled to the combined urea plant. However, this implies condensation of the off-gas into a solution containing NH₃ and CO₂ suitable to be conveyed back to the urea plant, and a related condensation section.

Moreover, in order to avoid ammonia losses from the absorber and minimize the carbon dioxide content in the melamine solution leaving the stripper, a sequence of desorption and absorption occurring, respectively, in the stripper and the subsequent absorber is strongly required. However, it implies a high steam consumption inside the stripper, which is very disadvantageous in terms of costs.

WO 2009/080176 discloses a non-catalytic process for the preparation of melamine wherein a gaseous mixture of vaporized melamine and reaction off-gases is quenched by contact with an aqueous ammonium carbamate solution.

### Summary of the invention

The purpose of the invention is to avoid the above drawbacks of the prior art.

Said purpose is achieved with a high pressure melamine plant and a related process according to the claims.

The term "high pressure melamine plant" denotes a plant for the synthesis of melamine using a high pressure non-catalytic process, which comprises a synthesis section operating at a high pressure, which is typically greater than 7 MPa, and a purification section operating at a lower pressure, which is typically 2 to 3 MPa.

The high pressure melamine plant according to the invention comprises a synthesis section and a melamine purification section, wherein said synthesis section provides a first melamine-containing stream, and said purification section comprises:
a quencher receiving said first melamine-containing stream from the synthesis section;
a stripper fed with a second melamine-containing stream from said quencher and with a stripping medium, said stripping medium being preferably steam, wherein purified melamine is obtained and vapours are extracted;
a heat exchanger, wherein said vapours are at least partially condensed by heat exchange with a cooling medium, obtaining a condensed stream;
an absorber fed with said condensed stream from said heat exchanger and providing an aqueous solution comprising ammonia and carbon dioxide, at least a portion of said aqueous solution being exported from the purification section of the plant. Preferably, said aqueous solution is at least partially recycled to a tank.

The vapours extracted from the stripper may contain ammonia and carbon dioxide.

The term "first melamine-containing stream" refers to a raw melamine melt and the term "second melamine-containing stream" refers to a melamine solution.

Advantageously, a first portion of the aqueous solution extracted from the absorber is recirculated inside the absorber itself, where it acts as absorbing medium for said condensed stream, and a second portion of said aqueous solution is exported from the purification section of the plant. More advantageously, said first portion is recirculated inside said absorber after passing through a cooler.

An aspect of the invention is the solution withdrawn from the absorber being a diluted solution, i.e. having a low content of ammonium carbamate. Preferably said solution has a water content of at least 60%.

According to a preferred embodiment the quenching medium is predominantly water or an aqueous solution of ammonia (ammonia-water). Preferably said quenching medium has a carbon dioxide content lower than 5% in weight, more preferably lower than 1%.

Preferably, a line is arranged to introduce a stream of water into the purification section of the melamine plant, a first portion of said stream of water forming said cooling medium for vapour condensation, and a second portion thereof bypassing the heat exchanger and being supplied to the quencher. Preferably said second portion of water is supplied directly to the quencher. Both said first portion of water (after a heat exchange) and said second portion of water act as quenching medium in the aforesaid quencher.

Preferably, said stream of water is a recycle stream coming from the plant. Said recycle stream may contain dissolved melamine, OATs, ammonia and carbon dioxide. The water content is preferably at least 90%, more preferably at least 95% end even more preferably is at least 98%. The carbon dioxide content of the aforesaid recycle stream is preferably lower than 5%, more preferably lower than 1%. The above percentages are in weight.

According to an advantageous embodiment of the invention, said first and second portions of the stream of water contain ammonia. Ammonia is advantageously added to said stream of water before the splitting into the above first and second portions.

Addition of ammonia to said stream of water is particularly preferred because ammonia is able to maintain an adequate pH value in the quencher and prevents the formation of oxi-amino-triazines (OATs).

According to preferred embodiments, the portion of water leaving the above heat exchanger is at least partially sent back to the quencher. According to even more preferred embodiments, said portion of water leaving the above heat exchanger is at least partially heated in a further heat exchanger and then supplied to the quencher.

The stripping of the second melamine-containing stream is preferably carried out before separation of solid melamine.

Preferably, said first melamine-containing stream is free, or substantially free, of melamine offgas. According to a particularly preferred embodiment, the synthesis section of the high pressure melamine plant comprises a melamine reactor, a stripping reactor and a scrubber.

The term of melamine offgas denotes the offgas released from the synthesis of melamine, predominantly made of carbon dioxide and ammonia. The reference to a stream which is free or substantially free of melamine offgas denotes that the stream contains no or a negligible amount of said offgas in the gaseous phase, since the offgas are removed in the synthesis section of the high pressure melamine plant.

The offgas-free melamine stream may contain a small amount of offgas dissolved in the liquid phase, particularly ammonia, since carbon dioxide is at least in part separated from liquid melamine in the stripping stage. A melamine-containing stream free from offgas is preferably generated by the above mentioned setup of a melamine reactor, stripping reactor and scrubber.

The very low amount or absence of offgas in the first melamine-containing stream, particularly the absence of carbon dioxide, reduces the duty and cost of the purification section. Accordingly, the purification section is aimed basically to quench and dilute the first melamine-containing stream and is not required to condense a large amount of gaseous ammonia and carbon dioxide.

Preferably, the scrubber is fed with at least part of the urea melt directed to the high pressure synthesis section. In the scrubber, the urea melt is preheated by using off-gas supplied by the melamine and/or stripping reactor as heat source. The melamine reactor is at least partially fed with the urea melt leaving the scrubber, providing raw melamine and separating off-gas. The second stripping reactor is fed with said raw melamine and gaseous ammonia as stripping agent, providing purified melamine and separating off-gas. At least a portion of the off-gas extracted from said melamine reactor and/or from said stripping reactor are fed to the scrubber, inside which the off-gas are brought into contact with the urea melt feed.

Even more preferably, said synthesis section also comprises a steam generator, which is supplied with a portion of the urea melt leaving the scrubber as heat source.

The term "purified melamine" refers to the melamine melt obtained in the stripping reactor, which has higher purity than the raw melamine obtained in the melamine reactor, but which needs be further purified in the subsequent purification section.

The term "high pressure section" denotes that the melamine reactor, the stripping reactor and the scrubber operate substantially at the same nominal pressure, said pressure being greater than 7 MPa and preferably greater than 10 MPa, for example 11 MPa.

It is also disclosed a revamping of the purification section of a prior art melamine plant comprising:
a quencher fed with a first melamine-containing stream collected from the synthesis section of the plant and an aqueous solution comprising ammonia and carbon dioxide, wherein melamine is dissolved and off-gas are extracted;
a stripper fed with a second melamine-containing stream from said quencher and with a stripping medium, said stripping medium being preferably steam, wherein purified melamine is obtained and vapours are extracted, said vapours being added with a stream of water once extracted from the stripper;
a first heat exchanger, wherein the vapours added with said stream of water are at least partially condensed by heat exchange with an aqueous solution comprising ammonia and carbon dioxide, providing a condensed stream;
an absorber fed with said condensed stream and a carbonate solution, which provides an aqueous solution comprising ammonia and carbon dioxide, said aqueous solution being at least partially used for condensing the vapours in said first heat exchanger, further heated in a second heat-exchanger and recycled to the quencher. Said carbonate solution is preferably recycled from an ammonia separation section.

Said method of revamping includes: installation of a line for at least partially feeding a stream of water (possibly added with ammonia) to the first heat exchanger for condensing said vapours; installation of a line for at least partially sending the stream of water leaving the first heat exchanger to the quencher; installation of a line for at least partially exporting the aqueous solution provided by said absorber from the purification section of the plant; discontinuing the line for feeding said aqueous solution to the first heat exchanger, for subsequent feeding to the second exchanger and recycle to the quencher; discontinuing the line for adding water to the vapours extracted from the stripper; discontinuing the line for introducing the carbonate solution into the absorber.

Said stream of water may be at least partially supplied to the first heat exchanger is a recycle stream coming from the plant. More preferably, a line is installed for injecting ammonia into said stream of water.

A line bypassing the first heat exchanger may be installed for supplying a portion of said stream of water, possibly added with ammonia, to the quencher. Said line can be installed downstream of the line for injecting ammonia into said stream of water, the ammonia being able to maintain an adequate pH value in the quencher preventing the formation of OATs.

The line of water leaving the first heat exchanger may passes through said second heat exchanger for further heating and then is-be supplied to the quencher.

A further portion of the aqueous solution extracted from the absorber can be directly supplied to the quencher and, during the revamping, said further line may be discontinued.

A line for extracting the off-gas from the quencher can be discontinued due to the lack of carbon dioxide and ammonia leaving the quencher as gaseous stream.

The above described purification section of the prior art may also be revamped by: installing a line feeding a stream of water to the quencher, at least a portion of said stream of water being heated inside said second heat exchanger before being sent to the quencher; dismissing the stripper; dismissing the first heat exchanger; redirecting the stripping medium to the quencher; installing a line for exporting said second melamine-containing stream from the purification section of the plant; dismissing the absorber. A line may be installed for injecting ammonia into said stream of water.

Said second melamine-containing stream from the quencher may be subjected to further purification treatment downstream said purification section.

A pump can be installed downstream of the quencher for suitably pressurizing said second melamine-containing stream before being subjected to said further purification.

The above methods of revamping may concern the purification section of a high pressure melamine plant of the prior art provided with a synthesis section comprising a melamine reactor, a stripping reactor and a scrubber, as disclosed in the patent application No. EP15154205.7.

The present invention has the following advantages.

First, the undesired sequence of desorption and absorption taking place, respectively, in the stripper and the absorber is avoided because the ammonia content in the absorber is minimized, as well as the carbon dioxide content in the effluent from the stripper. As a consequence, the steam consumption inside the stripper is significantly reduced or eliminated in the case the revamping provides for the stripper elimination.

A further advantage is that the liquid stream leaving the quencher is not saturated with ammonia and carbon dioxide, which provides for less off-gas extracted from the subsequent stripping and a lower content of ammonia and carbon dioxide in the aqueous solution collected from the absorber. An even lower content of ammonia and carbon dioxide in the liquid stream leaving the quencher justifies the dismissing of the stripper and the absorber.

Moreover, the quencher does not provide any gaseous stream and no off-gas are recovered therefrom. As a consequence, an off-gas condensation unit is no longer required.

Another advantage is that a portion of the aqueous solution from the absorber is exported from the purification section for further use in the process, thanks to the fact that the water input for the quencher is provided by a stream of water recycled from the plant.

The advantages will emerge even more clearly with the aid of the detailed description below relating to preferred embodiments.

### Description of the figures

Fig. 1 is a block scheme of the melamine plant according to a preferred embodiment of the invention.
Fig. 2 is a schematic diagram of the high-pressure section of the plant according to Fig. 1.
Fig. 3 is a block scheme of a melamine plant according to the prior art.
Fig. 4 is a block scheme of a melamine plant after revamping of the plant of Fig. 3, wherein the method of revamping is not part of the invention.

### Detailed description

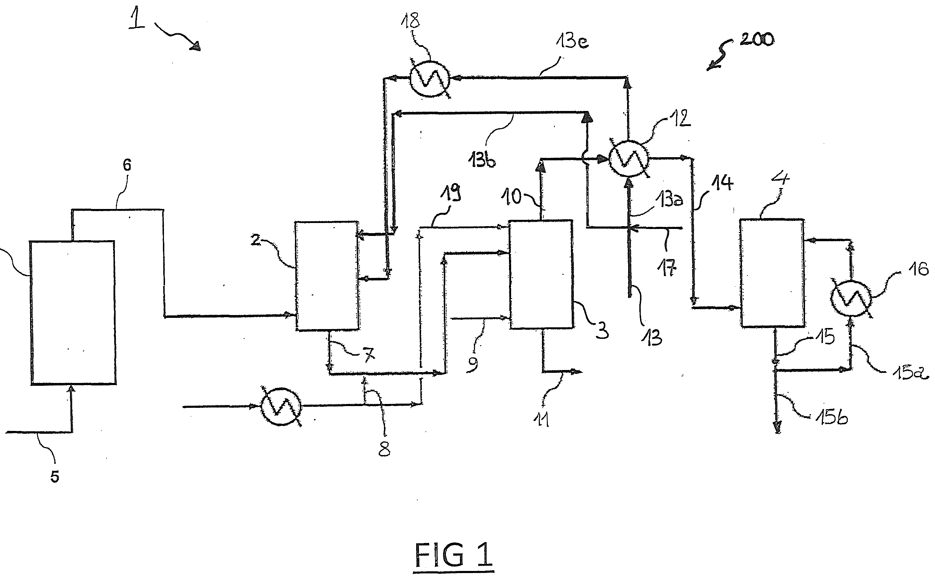

With reference to Fig. 1, the melamine plant 1 comprises a high-pressure section 100 and a purification section 200 which operates at significantly lower pressure and temperature.

The high-pressure section 100 is fed with urea melt 5 and produces melamine 6. As the melamine 6 leaves the high-pressure section 100, the pressure is lowered from above 7 MPa to 0.4-2 MPa and melamine is supplied to the purification section 200.

Said purification section 200 essentially comprises a quencher 2, a stripper 3 diluted with a stream of water 8 coming from the plant and fed to the stripper 3.

The stripper 3 is further fed with steam 9, which preferably comes from the plant, and with a further stream of water 19 coming from the plant. In the stripper 3, vapours 10 are extracted from the top and an ammonia- and carbon dioxide- free melamine solution 11 is collected from the bottom. Said solution 11 is then subjected to further purification, comprising for example: filtration, clarification with activated carbon, crystallization, solid separation of the melamine crystals and drying.

The vapours 10 are then sent to a heat exchanger 12, where they are condensed by thermal exchange with an aqueous solution of ammonia 13a.

The condensed vapours 14 are fed into the absorber 4, which provides an aqueous solution 15. Said aqueous solution is split into two portions: the first portion 15a is passed through a cooler 16 and recirculated inside the absorber 4 and the second portion 15b is exported from the purification section 200 and preferably recycled to the plant.

A stream of water 13 recycled from the plant and an ammonia stream 17 are mixed to provide an aqueous ammonia solution, which is split into portions 13a, 13b. The first portion 13a is fed to the above heat exchanger 12, where it is preheated to provide stream 13c, and the second portion 13b is supplied to the quencher 2.

Said stream 13c of preheated aqueous solution is fed to another heat exchanger 18, where it is further heated, and supplied to the quencher 2.

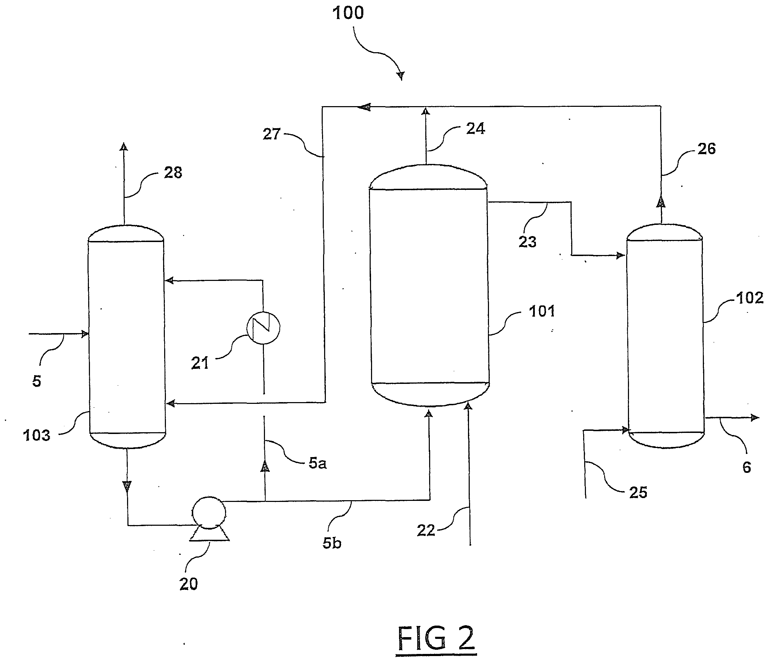

Fig. 2 shows the high-pressure section 100 of the plant 1 of Fig. 1 according to an embodiment of the invention, which comprises essentially a first melamine reactor 101, a second stripping reactor 102 and a scrubber 103.

In particular, the scrubber 103 is fed with the urea melt 5. The urea melt leaving the scrubber passes through a pump 20 and a first portion 5a of said urea melt is recirculated inside the scrubber 103 after passing through a heat exchanger 21, where it is advantageously cooled producing vapour, and a second portion 5b of said urea melt is supplied to the melamine reactor 101.

The melamine reactor 101 is also fed with a heat-carrier fluid 22 which supplies heat to the melamine reactor 101 in order to promote the endothermic reaction for conversion of urea into melamine. Typically said heat-carrier fluid 22 consists of molten salts which circulate inside suitable heating pipes of the reactor.

Raw melamine is separated as stream 23 and off-gas are extracted as stream 24.

Said raw melamine 23 is sent to the second stripping reactor 102, which is further fed with gaseous ammonia 25 as stripping agent. The purified melamine 6 leaving the stripping reactor 102 is fed to the quencher 2 of the purification section 200 of the plant, generally operating at a lower pressure than the synthesis section 100, and the off-gas 26 extracted therefrom are combined with the off-gas 24 from the melamine reactor 101, providing a stream 27.

Said stream 27 is introduced into the scrubber 103, where it undergoes washing with the urea melt streams 5 and 5a, respectively feeding and recirculating streams. A high pressure stream of anhydrous off-gas 28, mainly composed of ammonia and carbon dioxide, is extracted from the scrubber 103.

For example, the urea melt 5 is produced in a urea plant connected to the melamine plant comprising the section 100. The off-gas 28, in view of their content of NH3 and CO2 (which constitute the reagents for obtaining urea), are preferably recycled to said urea plant.

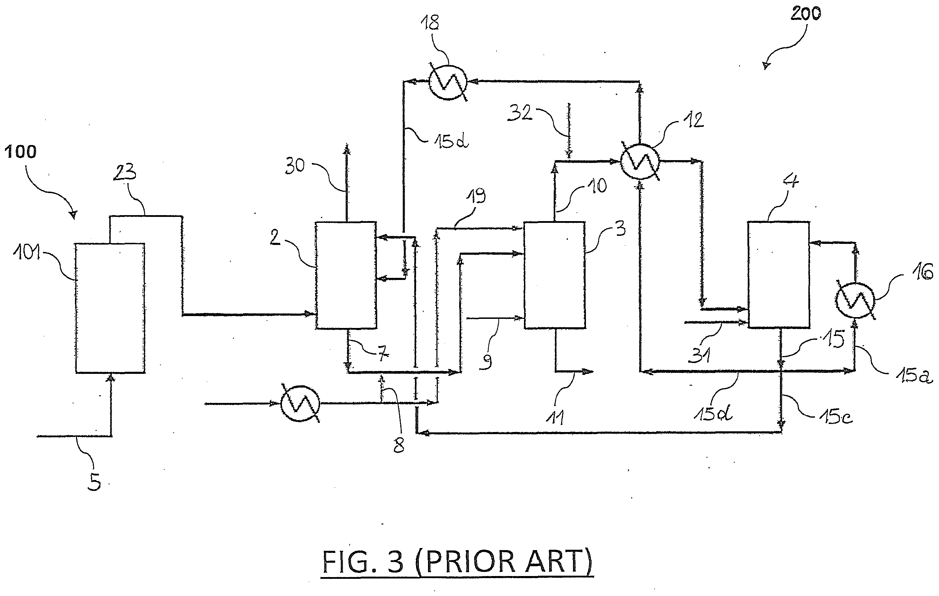

Fig. 3 shows a melamine plant according to the prior art, comprising a high-pressure section 100 and a purification section 200.

The high-pressure section 100 essentially comprises the melamine reactor 101, which is fed with the urea melt 5 and produces a stream 23 containing melamine and off-gas, which is supplied to the purification section 200.

The purification section 200 essentially comprises a quencher 2, a stripper 3 and an absorber 4. In the quencher 2, which is fed with said stream 23 melamine and off-gas, which is supplied to the purification section 200.

The purification section 200 essentially comprises a quencher 2, a stripper 3 and an absorber 4. In the quencher 2, which is fed with said stream 23 containing melamine and off-gas and an aqueous solution 15d comprising ammonia and carbon dioxide, melamine 7 is collected from the bottom and off-gas 30 are extracted from the top.

The off-gas 30 leaving the top of the quencher 2 are saturated with water and conveyed to a condensation section (not shown); melamine 7 is diluted with a stream of water 8 and fed to the stripper 3, which is supplied with steam 9 and a further stream of water 19.

Vapours 10, which contain ammonia and carbon dioxide, are removed from the top of the stripper 3 and an ammonia- and carbon dioxide- free melamine solution 11 is collected from the bottom thereof. Said solution 11 is then subjected to further purification.

The vapours 10 are added with an amount of water 32, preferably recycled from the plant, condensed in a heat exchanger 12 and then absorbed in water within the absorber 4.

Said absorber is also fed with a carbonate solution 31, providing an aqueous solution 15. Said aqueous solution 15 is split into three portions: the first portion 15a is passed through a cooler 16 and recirculated inside the absorber 4, the second portion is recycled to the quencher 2 through a first line 15c and the third portion is recycled to the quencher 2 through a second line 15d.

The first line 15c provides direct supply of the above solution to the quencher 2, while the second line 15d provides: passage of the solution through the above mentioned heat exchanger 12 for condensing the off-gas 10 extracted from the stripper 3; passage through a further heat exchanger 18 where it is further heated; supply to the quencher 2.

Said plant of the prior art can be revamped to provide the melamine plant illustrated in Fig. 1, by means of the following operations:
installation of a line 13 for recycling a stream of water from the plant;
installation of a line 17 for injecting ammonia into said stream of water;
installation of a line 13a for feeding a first portion of said stream of water containing ammonia to the first heat exchanger 12, wherein said stream of water 13a is preheated;
installation of a line 13b for feeding a second portion of said stream of water containing ammonia to the quencher 2;
installation of a line 13c for sending the preheated water to the second heat exchanger 18 and then to the quencher 2;
installation of a line 15b for at least partially exporting the aqueous solution provided by said absorber 4 from the purification section 200 of the plant;
discontinuing the line 15d for feeding said aqueous solution to the first heat exchanger 12, for subsequent feeding to the second-exchanger 18 and recycle to the quencher 2;
discontinuing the line 15c for directly supplying said aqueous solution to the quencher 2;
discontinuing the line 32 for adding said stream of water 32 to the vapours 10 extracted from the stripper 3;
discontinuing the line 31 for introducing the carbonate solution into the absorber 4;
discontinuing the line 30 for extracting the off-gas 30 from the quencher 2.

The revamping of the purification section 200 is can be carried out after revamping of the high pressure synthesis section to provide the synthesis section of Fig. 2.

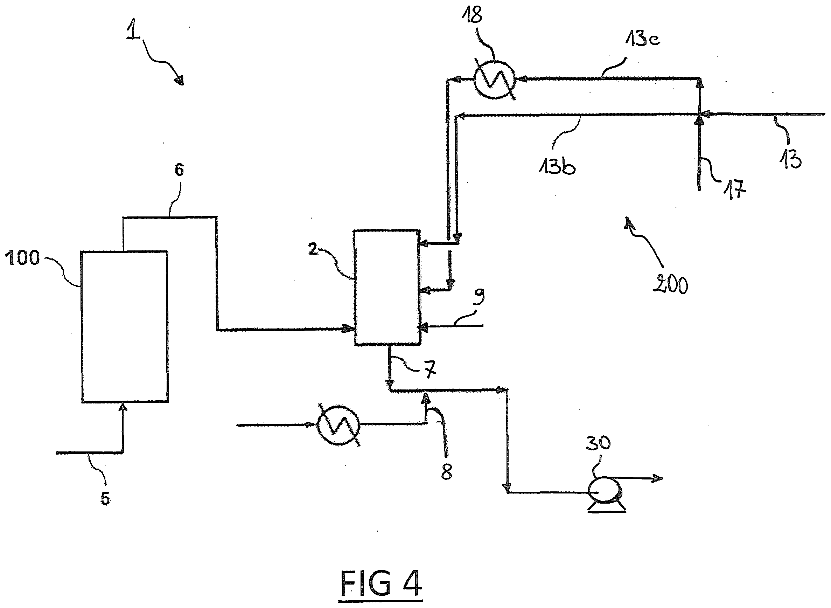

The plant of the prior art is may also be revamped to provide the melamine plant illustrated in Fig. 4, by means of the following operations:
redirecting the stripping medium (9) to the quencher (2);
installation of a line 13c for feeding a first portion of said stream of water containing ammonia to the second heat exchanger 18 and then to the quencher 2;
installation of a line 13b for feeding a second portion of said stream of water containing ammonia to the quencher 2;
installation of a line for exporting said second melamine-containing stream 7 from said quencher 2, said stream 7 being sent to a further purification section (not shown);
installation of a pump 30 downstream the quencher 2, which receives said second melamine-containing stream 7 before being sent to said further purification section;
dismissing the stripper 3, the first heat exchanger 12, the absorber 4 and the cooler 16;
discontinuing the inlet and outlet lines to / from the stripper 3, the first heat exchanger 12, the absorber 4 and the cooler 16;
discontinuing the line 30 for extracting the off-gas 30 from the quencher 2.

According to this embodiment, the revamping of the purification section 200 is carried out after revamping of the high pressure synthesis section to provide the synthesis section of Fig. 2.

## Claims

1. A high pressure melamine plant comprising a synthesis section (100) and a melamine purification section (200), wherein said synthesis section (100) provides a first melamine-containing stream (6), and said melamine purification section (200) comprises:
a quencher (2) receiving said first melamine-containing stream (6), which is a raw melamine melt, from the synthesis section (100);
a stripper (3) fed with a second melamine-containing stream (7) from said quencher (2) and also fed with a stripping medium (9), wherein purified melamine (11) is obtained and vapours (10) are extracted;
a heat exchanger (12), wherein said vapours (10) from the stripper (3) are at least partially condensed by heat exchange with a cooling medium (13a), obtaining a condensed stream (14);
an absorber (4) fed with said condensed stream (14) from said heat exchanger (12) and providing an aqueous solution (15) comprising ammonia and carbon dioxide, at least a portion (15b) of said aqueous solution being exported from the purification section (200) of the plant;
wherein said first melamine-containing stream (6) is free, or substantially free, of melamine offgas.

2. Plant according to claim 1, wherein a first portion (15a) of said aqueous solution (15) is recirculated inside the absorber (4) to act as absorbing medium for said condensed stream, and a second portion (15b) of said aqueous solution (15) is exported from the purification section (200) of the plant.

3. Plant according to claim 1 or 2, comprising a line (13) arranged to introduce a stream of water into the purification section (200), wherein: a first portion (13a) of said stream of water (13) forms said cooling medium for the at least partial condensation of said stripper vapours (10); a second portion (13b) of said stream of water (13) is directed to the quencher (2); said first portion of water (13a), after leaving said heat exchanger (12), and said second portion of water (13b) act as quenching medium of said first melamine-containing stream.

4. Plant according to claim 3, comprising a line (17) arranged to inject ammonia into said stream of water (13).

5. Plant according to any of the previous claims, wherein said cooling medium (13c) leaving said heat-exchanger (12) is at least partially supplied to the quencher (2), preferably after being heated in a further heat exchanger (18).

6. Plant according to any of the previous claims, wherein said synthesis section (100) comprises a first melamine reactor (101), a second stripping reactor (102) and a scrubber (103).

7. A process for purifying a melamine-containing stream (6) from the synthesis section (100) of a high pressure melamine plant, in a melamine purification section (200) of said plant, wherein said melamine-containing stream (6) is provided by said synthesis section (100) free, or substantially free, of melamine offgas and is subjected to a quenching process which dissolves melamine to provide a second melamine-containing stream (7); said second stream (7) is subjected to stripping with a stripping medium (9) which provides purified melamine (11) and vapours (10); said vapours (10) are at least partially condensed by heat exchange with a cooling medium (13a) providing a condensed stream (14); said condensed stream (14) is subjected to an absorption process providing an aqueous solution (15), which is at least partially (15b) exported from said purification section.

8. A process according to claim 7, wherein said quenching process is carried out with a quenching medium which has a carbon dioxide content lower than 5% in weight, more preferably lower than 1%.

9. Process according to any of claims 7 or 8, wherein a first portion (13a) of a stream of water (13) forms said cooling medium and a second portion (13b) of said stream of water (13) is directed to the quenching process.

10. Process according to claim 9, wherein ammonia (17) is injected into said stream of water (13).

11. Process according to any of claims 7 to 10, wherein said cooling medium (13c) used to condense the vapours (10) from the stripping process is directed to the quenching process, preferably after being further heated.

## Patentansprüche

1. Hochdruckmelaminanlage, umfassend einen Syntheseabschnitt (100) und einen Melaminreinigungsabschnitt (200), wobei der Syntheseabschnitt (100) einen ersten melaminhaltigen Strom (6) bereitstellt und der Melamin-Reinigungsabschnitt (200) umfasst: einen Quencher (2), der den ersten melaminhaltigen Strom (6), der eine rohe Melaminschmelze ist, aus dem Syntheseabschnitt (100) erhält; einen Stripper (3), der mit einem zweiten melaminhaltigen Strom (7) aus dem Quencher (2) gespeist und auch mit einem Strippingmittel (9) gespeist wird, wobei gereinigtes Melamin (11) erhalten wird und Dämpfe (10) extrahiert werden; einen Wärmetauscher (12), wobei die Dämpfe (10) aus dem Stripper (3) zumindest teilweise durch Wärmeaustausch mit einem Kühlmedium (13a) kondensiert werden, wodurch ein kondensierter Strom (14) erhalten wird; einen Absorber (4), der mit dem kondensierten Strom (14) von dem Wärmetauscher (12) gespeist wird und eine wässrige Lösung (15) bereitstellt, die Ammoniak und Kohlendioxid umfasst, wobei mindestens ein Teil (15b) der wässrigen Lösung aus dem Reinigungsabschnitt (200) der Anlage exportiert wird.

2. Anlage nach Anspruch 1, wobei ein erster Teil (15a) der wässrigen Lösung (15) innerhalb des Absorbers (4) zurückgeführt wird, um als Absorptionsmittel für den kondensierten Strom zu wirken, und ein zweiter Teil (15b) der wässrigen Lösung (15) aus dem Reinigungsabschnitt (200) der Anlage exportiert wird.

3. Anlage nach Anspruch 1 oder 2, umfassend eine Leitung (13), die angeordnet ist, um einen Wasserstrom in den Reinigungsabschnitt (200) einzuführen, wobei: ein erster Teil (13a) des Wasserstroms (13) das Kühlmittel für die zumindest partielle Kondensation der Stripper-Dämpfe (10) bildet; ein zweiter Teil (13b) des Wasserstroms (13) zum Quencher (2) geleitet wird; der erste Teil des Wassers (13a) nach dem Verlassen des Wärmetauschers (12) und der zweite Teil des Wassers (13b) als Quenchingmittel des ersten melaminhaltigen Stroms wirken.

4. Anlage nach Anspruch 3, umfassend eine Leitung (17), die angeordnet ist, um Ammoniak in den Wasserstrom (13) zu injizieren.

5. Anlage nach einem der vorhergehenden Ansprüche, wobei das Kühlmedium (13c), das den Wärmetauscher (12) verlässt, dem Quencher (2) zumindest teilweise zugeführt wird, vorzugsweise nachdem es in einem weiteren Wärmetauscher (18) erhitzt wurde.

6. Anlage nach einem der vorhergehenden Ansprüche, wobei der Syntheseabschnitt (100) einen ersten Melaminreaktor (101), einen zweiten Stripp-Reaktor (102) und einen Wäscher umfasst.

7. Verfahren zur Reinigung eines melaminhaltigen Stroms (6) aus dem Syntheseabschnitt (100) einer Hochdruckmelaminanlage in einem Melaminreinigungsabschnitt (200) der Anlage, wobei der melaminhaltige Strom (6) durch den Syntheseabschnitt (100) frei, oder im Wesentlichen frei, von Melaminabgasen bereitgestellt wird und einem Quenchingprozess unterzogen wird, welcher Melamin auflöst, um einen zweiten melaminhaltigen Strom (7) bereitzustellen; der zweite Strom (7) wird einem Strippingmittel (9) ausgesetzt, das gereinigtes Melamin (11) und Dämpfe (10) bereitstellt; die Dämpfe (10) werden zumindest teilweise durch Wärmeaustausch mit einem Kühlmedium (13a) kondensiert, wodurch ein kondensierter Strom (14) bereitgestellt wird; der kondensierte Strom (14) wird einem Absorptionsprozess unterzogen, wobei eine wässrige Lösung (15) bereitgestellt wird, die zumindest teilweise (15b) aus dem Reinigungsabschnitt exportiert wird.

8. Verfahren nach Anspruch 7, wobei der Quenchingprozess mit einem Quenchingmittel durchgeführt wird, das einen Kohlendioxidgehalt von weniger als 5 Gew.-%, bevorzugter weniger als 1%, aufweist.

9. Verfahren nach einem der Ansprüche 7 oder 8, wobei ein erster Teil (13a) eines Wasserstroms (13) das Kühlmittel bildet und ein zweiter Teil (13b) des Wasserstroms (13) zu dem Quenchingprozess geleitet wird.

10. Verfahren nach Anspruch 9, wobei Ammoniak (17) in den Wasserstrom (13) injiziert wird.

11. Verfahren nach einem der Ansprüche 7 bis 10, wobei das Kühlmittel (13c), das zum Kondensieren der Dämpfe (10) aus dem Stripppingrozess verwendet wird, zu dem Quenchingprozess geleitet wird, vorzugsweise nach weiterem Erhitzen.

## Revendications

1. Usine de mélamine haute pression comprenant une section de synthèse (100) et une section de purification de mélamine (200), dans laquelle ladite section de synthèse (100) fournit un premier courant contenant de la mélamine (6), et ladite section de purification de mélamine (200) comprend :
un désactivateur (2) recevant ledit premier courant contenant de la mélamine (6), qui est une masse fondue de mélamine brute, provenant de la section de synthèse (100) ;
un extracteur (3) alimenté par un deuxième courant contenant de la mélamine (7) provenant dudit désactivateur (2) et également alimenté par un milieu d'extraction (9), où de la mélamine purifiée (11) est obtenue et des vapeurs (10) sont extraites ;
un échangeur de chaleur (12), où lesdites vapeurs (10) provenant de l'extracteur (3) sont au moins partiellement condensées par échange de chaleur avec un milieu de refroidissement (13a), donnant un courant condensé (14) ;
un absorbeur (4) alimenté par ledit courant condensé (14) provenant dudit échangeur de chaleur (12) et fournissant une solution aqueuse (15) comprenant de l'ammoniac et du dioxyde de carbone, au moins une partie (15b) de ladite solution aqueuse étant exportée à partir de la section de purification (200) de l'usine ;
dans laquelle ledit premier courant contenant de la mélamine (6) est exempt, ou pratiquement exempt, de dégagement gazeux de mélamine.

2. Usine selon la revendication 1, dans laquelle une première partie (15a) de ladite solution aqueuse (15) est remise en circulation à l'intérieur de l'absorbeur (4) pour agir comme un milieu absorbant pour ledit courant condensé, et une deuxième partie (15b) de ladite solution aqueuse (15) est exportée à partir de la section de purification (200) de l'usine.

3. Usine selon la revendication 1 ou 2, comprenant une conduite (13) disposée de manière à introduire un courant d'eau dans la section de purification (200), dans laquelle : une première partie (13a) dudit courant d'eau (13) forme ledit milieu de refroidissement pour la condensation au moins partielle desdites vapeurs de l'extracteur (10) ; une deuxième partie (13b) dudit courant d'eau (13) est dirigée vers le désactivateur (2) ; ladite première partie d'eau (13a), après avoir quitté ledit échangeur de chaleur (12), et ladite deuxième partie d'eau (13b) agissent comme un milieu de désactivation dudit premier courant contenant de la mélamine.

4. Usine selon la revendication 3, comprenant une conduite (17) disposée de manière à injecter de l'ammoniac dans ledit courant d'eau (13).

5. Usine selon l'une quelconque des revendications précédentes, dans laquelle ledit milieu de refroidissement (13c) quittant ledit échangeur de chaleur (12) est au moins partiellement fourni au désactivateur (2), de préférence après avoir été chauffé dans un échangeur de chaleur (18) supplémentaire.

6. Usine selon l'une quelconque des revendications précédentes, dans laquelle ladite section de synthèse (100) comprend un premier réacteur de mélamine (101), un deuxième réacteur d'extraction (102) et un épurateur (103).

7. Procédé pour purifier un courant de la mélamine (6) provenant de la section de synthèse (100) d'une usine de mélamine haute pression, dans une section de purification de mélamine (200) de ladite usine, où ledit courant contenant de la mélamine (6) est fourni par ladite section de synthèse (100) en étant exempt, ou pratiquement exempt, de dégagement gazeux de mélamine et est soumis à un traitement de désactivation qui dissout la mélamine pour former un deuxième courant contenant de la mélamine (7) ; ledit deuxième courant (7) est soumis à une extraction avec un milieu d'extraction (9) qui donne de la mélamine purifiée (11) et des vapeurs (10) ; lesdites vapeurs (10) sont au moins partiellement condensées par échange de chaleur avec un milieu de refroidissement (13a) fournissant un courant condensé (14) ; ledit courant condensé (14) est soumis à un procédé d'absorption fournissant une solution aqueuse (15), qui est au moins partiellement (15b) exportée à partir de ladite section de purification.

8. Procédé selon la revendication 7, dans lequel ledit traitement de désactivation est mis en œuvre avec un milieu de désactivation qui a une teneur en dioxyde de carbone inférieure à 5 % en poids, plus préférablement inférieure à 1 %.

9. Procédé selon l'une quelconque des revendications 7 et 8, dans lequel une première partie (13a) d'un courant d'eau (13) forme ledit milieu de refroidissement et une deuxième partie (13b) dudit courant d'eau (13) est dirigée vers le traitement de désactivation.

10. Procédé selon la revendication 9, dans lequel de l'ammoniac (17) est injecté dans ledit courant d'eau (13).

11. Procédé selon l'une quelconque des revendications 7 à 10, dans lequel ledit milieu de refroidissement (13c) utilisé pour condenser les vapeurs (10) provenant du traitement d'extraction est dirigé vers le traitement de désactivation, de préférence après avoir été encore chauffé.
